# EUROPEAN PATENT SPECIFICATION

(11) **EP 3 372 155 B1**
(45) Date of publication and mention of the grant of the patent: **25.01.2023**
(21) Application number: 15907842.7
(22) Date of filing: 06.11.2015
(51) Int. Cl.: A61B 5/0245, A61B 5/00, A61B 5/024

(54) **MEAL TIME ESTIMATION METHOD, MEAL TIME ESTIMATION PROGRAM, AND MEAL TIME ESTIMATION DEVICE**
VERFAHREN ZUR SCHÄTZUNG DES ZEITPUNKTS EINER MAHLZEIT, PROGRAMM ZUR SCHÄTZUNG DES ZEITPUNKTS EINER MAHLZEIT UND VORRICHTUNG ZUR SCHÄTZUNG DES ZEITPUNKTS EINER MAHLZEIT
PROCÉDÉ D'ESTIMATION D'UNE HEURE DE REPAS, PROGRAMME D'ESTIMATION D'UNE HEURE DE REPAS ET DISPOSITIF D'ESTIMATION D'UNE HEURE DE REPAS

(43) Date of publication of application: 12.09.2018
(73) Proprietor: Fujitsu Limited, Kawasaki-shi, Kanagawa 211-8588 (JP)
(72) Inventor: MAEDA, Kazuho, Kawasaki-shi Kanagawa 211-8588 (JP); MORI, Tatsuya, Kawasaki-shi Kanagawa 211-8588 (JP); UCHIDA, Daisuke, Kawasaki-shi Kanagawa 211-8588 (JP); INOMATA, Akihiro, Kawasaki-shi Kanagawa 211-8588 (JP)
(74) Representative: Haseltine Lake Kempner LLP
(86) International application number: PCT/JP2015/081415
(87) International publication number: WO 2017/077656

(56) References cited:
- JP-A- H10 504 739
- JP-A- 2001 327 472
- JP-A- 2003 173 375
- JP-A- 2004 515 291
- JP-A- 2005 021 450
- JP-A- 2007 048 180
- JP-A- 2012 045 191

## Description

### TECHNICAL FIELD

The embodiments discussed herein are related to an information processing apparatus, a method and a program.

### BACKGROUND ART

Healthcare such as prophylaxis of lifestyle-related diseases, diet, medical service, or the like including metabolic syndrome and diabetes has attracted attention. In a case where such healthcare is realized, a process of recognizing a problem in their lifestyle habits and improving the problem by recording the lifestyle habits such as daily exercise and meals.

For example, as a precautionary measure concerning "meal", as described below, a method of controlling meals such as "when", "what", "how much" is included. Specifically, there are items such as regular eating three meals (when), taking breakfast (when), balancing nutrition (what), not taking too much calories (how much), and refraining from salt (what).

Here, for example, when there is a record of "when" you eat, it is possible to implement a healthcare service such as detecting irregular eating habits and providing advice on preventive measures.

For example, as an example of a technique for performing meal determination, an eating behavior detection system, an utterance and food and drink state detection system, a meal behavior detection device, and the like are proposed. For example, in the eating behavior detection system, eating determination is performed by detecting an action of rising and lowering an arm at the time of ingesting food using an acceleration sensor. In addition, in the utterance and food and drink state detection system, a frequency pattern peculiar to chewing of internal sound is detected using chewing when eating an object. In addition, in a case of the meal behavior detection device, under a condition that an infrared sensor is installed on a table or the like, threshold processing is performed to determine whether a human body is moving frequently after detecting the human body near the table.

However, in any of these techniques, in order to estimate meal behavior, there are limitations on how to eat or restriction on a place to estimate the meal behavior. Therefore, there is a lack of versatility. For example, a tendency of acceleration assumed by the eating behavior detection system corresponds only to one aspect of the arm's movement performed at the time of ingesting food, and in a case where the other arm is moved, the tendency of acceleration differs. Therefore, detection failure occurs. In addition, in a case of the utterance and food and drink state detection system, since the microphone is attached to around the neck at the time of meal, a burden is imposed on the body and the appearance is also deteriorated. In addition, in a case of the meal behavior detection device, it is only possible to recognize the meal in the fixed environment, such as the place where the infrared sensor is installed.

In addition, as an example of a technique using a pulse wave for the meal determination, a living management terminal device is also proposed. In the living management terminal device, in addition to the appearance of the chewing feature occurring at the time of the meal, in a case where pulse rate rises and skin conductivity does not suddenly rise, it is determined that it is in the middle of the meal. As another example, the following lifestyle evaluation method is also proposed. In the lifestyle evaluation method, in a case where there is no big change in the body motion detected by the acceleration sensor, the heart rate measured by the heart rate sensor increases, an LF and HF decreases, and the HF increases, it is determined that eating is started. Conversely, in the lifestyle evaluation method, in a case where the heart rate measured by the heart rate sensor decreases, the LF and HF increases and the HF decreases, it is determined that eating is ended. Japanese Laid-open Patent Publication Nos. 2000-245713, 2003-173375, 2004-81471, and 2007-48180, Japanese National Publication of International Patent Application No. 10-504739, Japanese Laid-open Patent Publication Nos. 2011-115508, 2008-61790, and 2010-158267 are examples of relevant prior art documents which relate to meal time estimation.

### SUMMARY

### TECHNICAL PROBLEM

However, in the above technique, when the labeled supervised data is not applied, estimation accuracy of the meal time may be lowered in some cases.

That is, there are individual differences in a pattern of increasing in the heart rate accompanying the meal. In order to reduce an influence of individual differences and to estimate the meal time from the heart rate, pulse rate, or the like, there is one aspect where utilization of the labeled supervised data is inevitable. For example, in the above-described living management terminal device, when there is no supervised data such as the pulse rate and the skin conductivity at the time of meal, it is difficult to reduce the influence of the individual differences from an estimation result of the meal time. In addition, in the lifestyle evaluation method described above, there is no supervised data such as heart rate, the LF and HF, and the HF at the time of meal, it is difficult to reduce the influence of the individual differences from the estimation result of the meal time.

In one aspect, an object is to provide a meal time estimation method, a meal time estimation program, and a meal time estimation device capable of suppressing deterioration of estimation accuracy of meal time.

### SOLUTION TO PROBLEM

The invention is defined in the appended claims.

### ADVANTAGEOUS EFFECTS OF INVENTION

It is possible to suppress the deterioration of the estimation accuracy of the meal time.

### BRIEF DESCRIPTION OF DRAWINGS

FIG. 1 is a diagram illustrating a configuration of a healthcare supporting system according to Example 1;
FIG. 2 is a diagram illustrating an example of heart rate data;
FIG. 3 is a diagram illustrating an example of a relationship between a peak time zone, a first peak, and a second peak;
FIG. 4 is a diagram illustrating an extraction example of the peak time zone;
FIG. 5 is a flowchart illustrating a procedure of meal time estimation processing according to Example 1;
FIG. 6 is a diagram illustrating an example of the heart rate data;
FIG. 7 is a diagram illustrating an example of a method for estimating the meal time by machine learning, this method does not fall within the scope of the claimed invention; and
FIG. 8 is a diagram illustrating a hardware configuration example of a computer that executes meal time estimation program according to Example 1 and Example 2 (wherein Example 2 does not fall within the scope of the claimed invention).

### DESCRIPTION OF EMBODIMENTS

### [Example 1]

### [System Configuration]

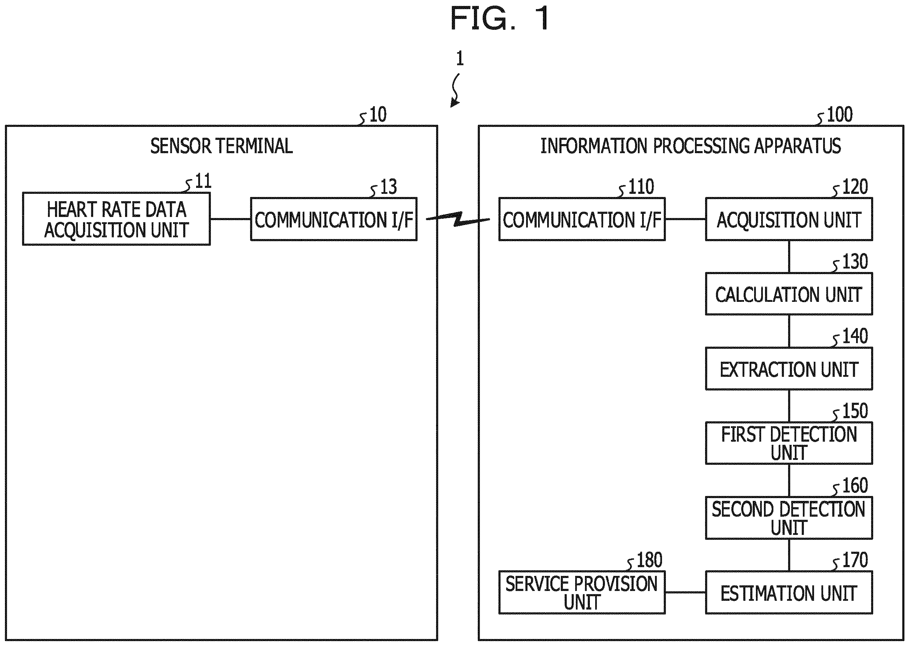

FIG. 1 is a diagram illustrating a configuration of a healthcare supporting system according to Example 1. A healthcare supporting system 1 illustrated in FIG. 1 provides various healthcare supporting services. For example, as an example of a healthcare supporting service, a service for recording living activities of a user of a sensor terminal 10 using sensing data collected by the sensor terminal 10, a service for recording the meal time, a derived service using the record, and the like are included.

As part of the healthcare supporting service, the healthcare supporting system 1 utilizes the knowledge that the second peak appears after the first peak appears after the meal and the second peak tends to be longer over the first peak for the meal time estimation.

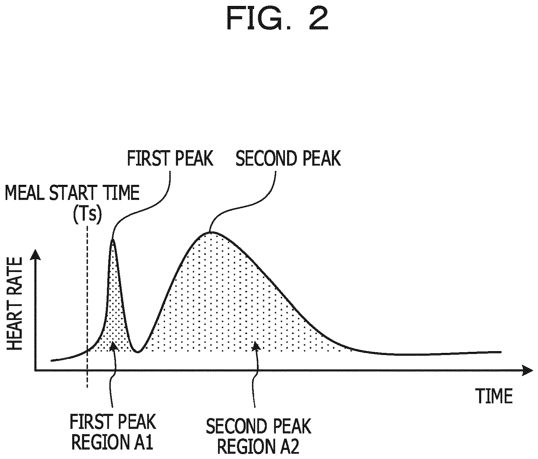

FIG. 2 is a diagram illustrating an example of the heart rate data. In FIG. 2, as an example, changes in the heart rate before and after the start of the meal are graphed. A vertical axis illustrated in FIG. 2 represents the heart rate per unit time, and a horizontal axis represents an elapsed time (time) from immediately before the meal starts.

As illustrated in FIG. 2, there are two peaks of which the heart rate rises (increases) with time and falls (decreases) as the heart rate changes occurring after the meal starts with the meal. That is, with the lapse of time from a meal start time Ts, a "first peak" which is the peak of the heart rate change appearing preceding after the meal starts and a "second peak" which is the peak of the heart rate change appearing following the first peak appear. In the following description, a predetermined region including a waveform of the first peak portion will be referred to as a "first peak region A1", and the predetermined region including the waveform of the second peak portion will be referred to as a "second peak region A2" in some cases.

Among them, the "first peak" is a rise in heart rate accompanying a meal action, for example, the first peak is estimated to be an increase in the heart rate due to peristaltic movement of the esophagus. In addition, the "second peak" is estimated to be an increase in the heart rate due to digestive activity in digestive organs (gastrointestine or the like) for ingestion ingested by meal action, that is, food or the like.

In this regard, it is possible to obtain the finding that after the meal, the second peak appears after the first peak appears and the second peak tends to be longer than the first peak over a long period of time.

Under such founding, the healthcare supporting system 1 realizes meal time estimation processing for detecting the first peak from the second peak of the time series data of each heart rate overlapping with the peak time zone appearing in the waveform of the average heart rate obtained by averaging the plurality of time series data pieces of the heart rate for each time and estimating the meal time. Therefore, as one aspect, it is possible to suppress a decrease in estimation accuracy of the meal time even when supervised data is not applied as an input. Hereinafter, the time series data of the heart rate may be referred to as the "heart rate data" in some cases.

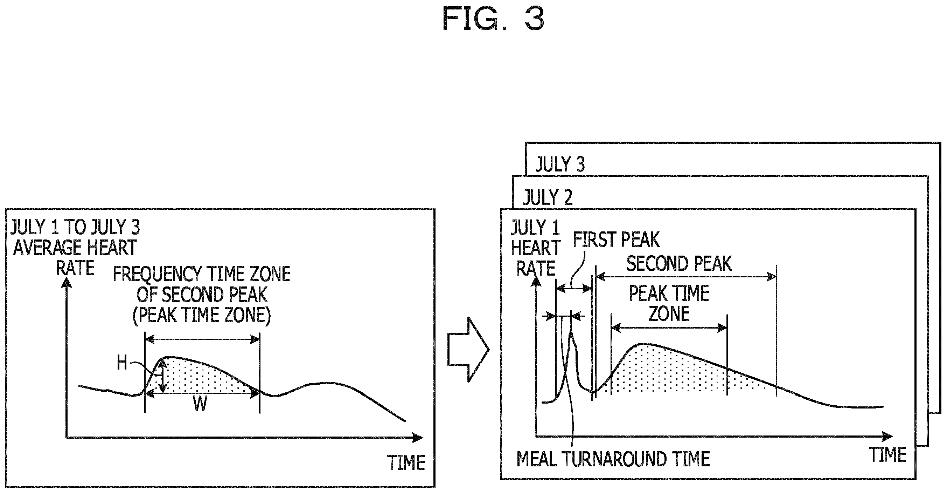

FIG. 3 is a diagram illustrating an example of a relationship between the peak time zone, the first peak, and the second peak. In the left part of FIG. 3, as an example, the waveform of the average heart rate which is obtained by averaging the heart rates at the same time among heart rate data pieces for three days from July 1 to July 3 measured for the same user is illustrated. In addition, in the right part of FIG. 3, the heart rate data of July 1 to July 3 are arranged, and heart rate data of July 1 is illustrated on behalf of these data pieces. In addition, the vertical axis illustrated in FIG. 3 represents the average heart rate or heart rate per unit time, and the horizontal axis represents a time.

As can be seen from the waveform of the average heart rate illustrated in the left part of FIG. 3, when heart rate data for three days are averaged for each time, the average heart rate includes the second peak of each heart rate data for three days overlap appears. This may be based on the fact that even when the same user has some variation, certain habits, such as the tendency of the meals to be taken according to the habit of taking meals during the lunch break, the fact that the second peak is longer than the first peak and the overlapping portion becomes longer between the heart rate data in general regardless of the variation in the meal time, or the like. As described above, a time zone in which an increasing width H of the heart rate and a rising period W of the heart rate are both equal to or more than the predetermined threshold value is extracted from the waveform of the average heart rate. Here, since the time zone extracted from the waveform of the average heart rate can be regarded as a time zone in which the second peak frequently occurs in each heart rate data on one aspect, it is described below as "peak time zone" in some cases.

When the peak time zone can be obtained in this manner, the second peak, the first peak, and thus the meal time can be estimated from each heart rate data. For example, as illustrated in the right part of FIG. 3, the second peak including the above peak time zone is detected for each heart rate data, and the first peak existing within a predetermined period, for example, one hour backward from the occurrence time of the second peak is further detected. The start time of the first peak, for example, the time at which the lowest value of the heart rate first appears before the first peak is estimated as the "meal start time", and the time at which the heart rate of the first peak is measured is defined as "meal end time". In addition, it is possible to further estimate the "meal turnaround time" by calculating the difference from the "meal start time" and the "meal end time". In this manner, at least any one of the "meal start time", the "meal end time", and the "meal turnaround time" or a combination thereof can be estimated as the "meal time".

As illustrated in FIG. 1, the healthcare supporting system 1 includes the sensor terminal 10 and an information processing device 100. Although FIG. 1 illustrates a case where there is one sensor terminal 10, a plurality of sensor terminals may be accommodated in the healthcare supporting system 1.

The sensor terminal 10 and the information processing device 100 are coupled to each other so as to communicate with each other. Here, as an example, a case where the sensor terminal 10 and the information processing device 100 are coupled by near field communication such as Bluetooth (registered trademark) low energy (BLE) or the like is assumed. However, it is possible to couple the sensor terminal 10 and the information processing device 100 each other via a certain network. For example, the sensor terminal 10 and the information processing device 100 can be coupled to each other via a local communication network such as a local area network (LAN) or a virtual private network (VPN), and a certain type of communication network such as the Internet.

The sensor terminal 10 is a terminal device on which a sensor is mounted.

As an embodiment, the sensor terminal 10 may be a terminal device dedicated to healthcare, a general wearable gadget such as smart glass or smart watch, or the like.

At least the heart rate sensor is mounted on the sensor terminal 10. The sensor terminal 10 detects, for example, the heart rate per unit time of the user who uses the sensor terminal 10 using this heart rate sensor. The heart rate data sensed using the heart rate sensor as described above is used for estimating the meal time. Although a case in which a heart rate sensor is mounted is exemplified here, the sensor that can be mounted is not limited to a heart rate sensor, and it does not reduce a possibility that other sensors are mounted thereon. For example, in a case where a motion sensor such as an acceleration sensor or a gyro sensor is mounted on the sensor terminal 10, it can be used to estimate the meal time using the sensing result to exclude exercise, such as walking, rising and falling and rising period of the heart rate accompanying the running, from the heart rate data.

In a case where the heart rate sensor is mounted on the sensor terminal 10, a wearable heart rate sensor to be worn on a living body part of the user, for example, around a chest, an arm, a wrist, or the like can be adopted. For example, a pulse by a photoelectric pulse wave sensor can be adopted. In this case, when the heart rate sensor can be mounted exclusively for healthcare, in a case where the wearable gadget includes a heart rate sensor, the heart rate sensor can also be diverted. In addition, as the heart rate sensor, it is not entirely desire to adopt a wearable type sensor. For example, it may be realize the detection of the heart rate in a non-contact state with the body part of the user by detecting the heart rate from a time series change in luminance with respect to an image in which a part of the user's body is imaged at a predetermined sampling frequency, or detecting a Doppler frequency accompanying the beat using a radio frequency (RF) motion sensor.

The "heart rate" referred to here is an index representing the number of beats of the heart which sends out blood, and its sensing method is a method of measuring the electrical activity of the heart, and may be a method of measuring the flow of blood. That is, the heart rate sensor may not be entirely mounted for the heart rate detection, and an electrocardiographic sensor for detecting an electrocardiographic signal may be mounted on the sensor terminal 10.

The heart rate data sensed by the sensor terminal 10 as described above is transmitted to the information processing device 100 in a state associated with identification information of the user, for example, a machine name and serial number of the sensor terminal 10. At this time, the heart rate data may be transmitted in real time every time when the heart rate is sensed, or the heart rate data pieces may be accumulated for a predetermined period of time, for example, 12 hours, 1 day, 1 week, 1 month, or the like and then the accumulated data pieces may be transmitted. In this example, in a case where the heart rate data is transmitted from the sensor terminal 10 to the information processing device 100 is exemplified. However, it may also possible to cause the sensor terminal 10 to extract the waveform of the average heart rate used for estimating the meal time from the heart rate data.

The information processing device 100 is a computer that provides the above-described healthcare supporting service. As the information processing device 100, it is possible to adopt a whole computer including a portable terminal device, a computer including a stationary type or a notebook type personal computer in general. The portable terminal device above described includes not only a mobile communication terminal such as a smartphone, a mobile phone, a personal handyphone system (PHS), but also a tablet terminal, a slate terminal, and the like.

In one embodiment, the information processing device 100 can be installed by installing a meal time estimation program that realizes the above-described healthcare supporting service as package software or online software on a desired computer. For example, the information processing device 100 estimates the meal time of the user of the sensor terminal 10 using the heart rate data received from the sensor terminal 10. In addition, the information processing device 100 can record the meal time, output a list of meal time zones ranging from a meal time recorded up to that time to a predetermined period, for example, one week, or the like. Alternatively, it is possible to analyze eating habits or diet from the meal time recorded so far and output various advices. For example, the above various types of information items can be outputted through an output device such as a display device, an audio output device, or a printing device of the information processing device 100. In addition, the output destination of the information is not entirely limited to the information processing device 100, and the output destination of the information may be another terminal apparatus used by the user. The output destination can be the terminal to be used by a person involved such as a relative of the user, a person in charge of medical care or nursing care. Accordingly, the above-described healthcare supporting service is realized.

### [Configuration of Sensor Terminal 10]

Next, a functional configuration of the sensor terminal 10 according to the present embodiment will be described. As illustrated in FIG. 1, the sensor terminal 10 includes a heart rate data acquisition unit 11 and a communication interface (I/F) unit 13. The sensor terminal 10 may include a functional unit of a known computer other than the functional unit illustrated in FIG. 1. For example, in a case where a terminal device dedicated to healthcare, the wearable gadget, or the portable terminal device is executed as the sensor terminal 10, hardware and software standard equipment of these devices can be installed.

The heart rate data acquisition unit 11 is a processing unit that acquires the heart rate data.

In one embodiment, the heart rate data acquisition unit 11 controls a heart rate sensor (not illustrated) to cause the heart rate sensor to sense the heart rate at a predetermined sampling cycle. As a result, the heart rate data acquisition unit 11 acquires time series data of the heart rate sensed by the heart rate sensor for each sampling point as heart rate data. For such heart rate data, for example, data in which items such as time and heart rate are associated can be adopted. Here, the "time" may be a system time locally managed on the sensor terminal 10, for example, an elapsed time from an arbitrary starting point, or may be a time represented by a calendar on a calendar such as year, month, day, hour, minute, or second. The "Heart rate" is expressed as the heart rate per unit time. For example, in a case where the unit time is 1 minute, the heart rate is expressed by beats per minute (bpm) or the like. In addition, in a case where the unit time is 1 second, the heart rate is expressed in Hz. Furthermore, when it is an index correlated with the "heart rate", it does not have to be the heart rate itself. For example, an RR interval of an electrocardiogram waveform in the electrocardiogram waveform is expressed in milliseconds and can be used instead of heart rate.

Here, the purpose of acquiring the heart rate by the sensor terminal 10 is to capture a response of a cardiovascular device accompanying the meal and use the response for the estimation of the meal time, and in a case where an index correlating with heart rate can be obtained from information obtained from electrocardiogram waveform and pulse wave waveform and information on blood flow rate in addition to the heart rate, the index can be used.

The communication I/F unit 13 is an interface that performs communication control with another device, for example, the information processing device 100 or the like.

As one embodiment, in a case where the sensor terminal 10 and the information processing device 100 are coupled to each other by the near field communication, a BLE module or the like can be adopted as the communication I/F unit 13. In addition, in a case where the sensor terminal 10 and the information processing device 100 are coupled to each other by a wireless communication network such as a LAN or a VLAN, a network interface card such as a LAN card can be adopted as the communication I/F unit 13. For example, the communication I/F unit 13 transmits the above-described heart rate data and the like to the information processing device 100. The communication I/F unit 13 receives an instruction for uploading the heart rate data and the like to the information processing device 100, an instruction on an interval for uploading heart rate data to the information processing device 100, or the like, an estimation result of the meal time, a diagnostic result using the estimation result, and the like from the information processing device 100.

As described above, the heart rate data detected by the heart rate data acquisition unit 11 is transmitted to the information processing device 100 by the communication I/F unit 13 according to an instruction from a control unit such as a central processing unit (CPU) or a micro-processing unit (MPU) (not-illustrated). At this time, every time when the heart rate is sampled, an amplitude of the heart rate may be transmitted to the information processing device 100 or may be transmitted to the information processing device 100 after accumulating the heart rate data pieces to a memory (not illustrated) over a predetermined period of time, for example, 12 hours, 1 day, 1 week, 1 month, or the like.

Various types of semiconductor memory devices, for example, a random access memory (RAM) and a flash memory can be adopted as a main storage device used by the processing unit such as the above heart rate data acquisition unit 11. In addition, the storage device referred to by each of the processing units described above is not entirely the main storage device and may be an auxiliary storage device. In this case, a hard disk drive (HDD), an optical disc, a solid state drive (SSD), or the like can be adopted.

### [Configuration of information processing device 100]

Next, the functional configuration of the information processing device 100 according to this embodiment will be described. As illustrated in FIG. 1, the information processing device 100 includes a communication I/F unit 110, an acquisition unit 120, a calculation unit 130, an extraction unit 140, a first detection unit 150, a second detection unit 160, an estimation unit 170, and a service provision unit 180. The information processing device 100 may include a functional unit of a known computer other than the functional unit illustrated in FIG. 1, for example, various types of an input and output devices and the like.

The communication I/F unit 110 is an interface that performs communication control with another device, for example, the sensor terminal 10 or the like.

As one embodiment, in a case where the sensor terminal 10 and the information processing device 100 are coupled to each other by the near field communication, the BLE module or the like can be adopted as the communication I/F unit 110. In addition, in a case where the sensor terminal 10 and the information processing device 100 are coupled to each other by a wireless communication network such as a LAN or a VLAN, a network interface card such as a LAN card can be adopted as the communication I/F unit 110. For example, the communication I/F unit 110 receives the heart rate data and the like from the sensor terminal 10. The communication I/F unit 110 receives an instruction for uploading the above-described heart rate data to the sensor terminal 10, an instruction on the interval for uploading the heart rate data to the information processing device 100 by the sensor terminal 10, an estimation result of the meal time, a diagnostic result using the estimation result, and the like to the sensor terminal 10.

The acquisition unit 120 is a processing unit that acquires the above-described heart rate data.

In one embodiment, the acquisition unit 120 can acquire the heart rate data from the sensor terminal 10 through the near field communication. In addition to accessing by such communication, the acquisition unit 120 reads the heart rate data stored in the auxiliary storage device such as the hard disk, the optical disc, or a removable medium such as a memory card or a Universal Serial Bus (USB) memory to acquire the heart rate data. In this example, the heart rate data is acquired from the sensor terminal 10 by the near field communication. However, in a case where the information processing device 100 includes a heart rate sensor or the like, the heart rate data output from the heart rate sensor may be acquired without any change.

Here, for estimating the meal time, in order to obtain the waveform of the average heart rate, a plurality of heart rate data pieces are acquired. Each heart rate data does not entirely have to be the same length of time. However, as the heart rate data in which the times when the heart rate is measured between the mutual data pieces are overlapped is collected, the accuracy of the meal time estimation can be expected to improve. In addition, each heart rate data can be set to an arbitrary time length. In the following, as an example, assuming a case where each heart rate data is daily measurement data, a situation where the heart rate data for a predetermined number of days is acquired on the condition that heart rate data for a predetermined number of days is accumulated will be exemplified.

The calculation unit 130 is a processing unit that calculates a statistical value of the heart rate for each time between the plurality of heart rate data pieces.

Once a plurality of heart rate data pieces are acquired by the acquisition unit 120, the calculation unit 130 executes predetermined statistical processing between heart rates measured at the same time among each heart rate data Thereby calculating the statistical heart rate for each time. As an example of such statistical processing, the calculation unit 130 can calculate an arithmetic average, a weighted average, a median, a mode, or the like. Therefore, the statistical heart rate data in which the statistical values of the heart rate measured at the same time during a predetermined number of days are aligned over time. In the context of the claimed invention, the average heart rate data is obtained.

Here, in a case of calculating the average heart rate data, the calculation unit 130 does not necessarily entirely use all the heart rate data pieces acquired by the acquisition unit 120. For example, it is possible to filter the heart rate data acquired by the acquisition unit 120 according to standard exemplified below. For example, the calculation unit 130 can extract sets of heart rate data pieces of dates having similar waveform shapes, and calculate average heart rate data from heart rate data of those dates. Whether or not the shapes of the waveforms are similar can be determined by performing threshold processing on the similarity such as the Euclidean distance and correlation coefficient calculated between the waveforms. In addition, the calculation unit 130 extracts the sets of dates of which the statistics of the heart rate data of a day, for example, average, standard deviation, and variance are similar to each other, and calculates the average heart rate data from the heart rate data of those dates. In addition, the calculation unit 130 may extract the sets of dates regarding the heart rate data, for example, dates whose days of the week, weekdays, and holidays coincide with each other and calculate average heart rate data from heart rate data of those dates. In addition, the calculation unit 130 can also extract the sets of dates acquired for each date, for example, dates having similar weather and temperature, and calculate average heart rate data from heart rate data of those dates. In addition, the calculation unit 130 may extract sensor information acquired from the user for each date, for example, the sets of dates of which body temperature and activity amount are similar to each other, and calculate the average heart rate data from the heart rate data of those dates.

The extraction unit 140 is a processing unit that extracts a peak time zone from the statistical heart rate data.

The extraction unit 140 extracts the time zone of the section in which the increasing width H of the heart rate and the rising period W of the heart rate satisfy the predetermined condition from the waveform of the average heart rate data calculated by the calculation unit 130 as the peak time zone. A first threshold value Th_{H} is set as the threshold value to be compared with the increasing width H of the heart rate, and a second threshold value Thw is set as the threshold value to be compared with the rising period W of the heart rate. Under such condition setting, the extraction unit 140 determines that the heart rate increasing width H on the waveform of the average heart rate data is equal to or greater than the first threshold value Th_{H} and extracts the section that the heart rate rising period W is equal to or larger than the second threshold value Thw. Therefore, the peak time zones are extracted from the waveform of the statistical heart rate data.

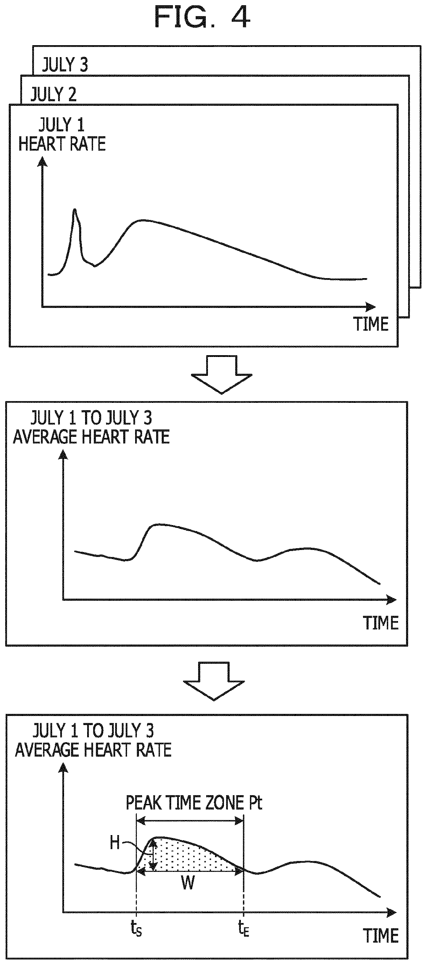

FIG. 4 is a diagram illustrating an extraction example of the peak time zone. In the upper portion of FIG. 4, the section of a part of the time zone of 3 days heart rate data measured from July 1 to July 3 measured for the same user is illustrated in an extractive manner. In the middle portion of FIG. 4, the waveform of the average heart rate data calculated from the heart rate data for 3 days illustrated in the upper portion of FIG. 4 is illustrated. Furthermore, in the lower portion of FIG. 4, a peak time zone Pt extracted from the waveform of the average heart rate data illustrated in the middle portion of FIG. 4 is illustrated. The vertical axis of each graph illustrated in FIG. 4 represents the heart rate or average heart rate per unit time, and the horizontal axis represents the time.

As illustrated in FIG. 4, the heart rate values at the same time are averaged among heart rate data pieces for 3 days illustrated in the upper portion of FIG. 4, thereby obtaining the waveform of the average heart rate data illustrated in the middle portion of FIG. 4. The following differences and similarities appear between the individual heart rate data before statistical processing and the average heart rate data after statistical processing. That is, in the individual heart rate data before the statistical processing, the increase in the heart rate data due to the first peak and exercise appears in the waveform on the waveform. However, as the heart rate increased due to the first peak and exercise becomes dull due to smoothing by statistical processing, it becomes finer or disappears. While there are such differences, the second peak continues for a longer period of time than the first peak. Therefore, even when there is some deviation in the actual meal time among the individual heart rate data, overlap of the second peak appears in the average heart rate data.

Thereafter, a peak time zone Pt illustrated in the lower portion of FIG. 4 is extracted from the waveform of the average heart rate data illustrated in the middle portion of FIG. 4. At the start time of the peak time zone, a time ts at which the minimum value is first detected tracing back from the time when the maximum value is measured is set at the section where the increasing width H of the heart rate on the waveform of the average heart rate data is equal to or greater than the first threshold value Th_{H} and the rising period W of the heart rate is equal to or greater than the second threshold value Thw. On the other hand, a time t_{E} at which the average heart rate decreases (recovers) to the same value as the average heart rate measured at the start time of the peak time zone in time elapse from the time when the maximum value is measured by the above conditions are satisfied section is set at the end time of the peak time zone. Therefore, a peak time zone Pt is defined from the waveform of the statistical heart rate data.

Here, for the sake of convenience of explanation, in the graphs of the upper portion to the lower portion of FIG. 4, the average heart rate data is illustrated in a state in which a part of the time zone of one day is extracted. However, this is not intended to limit that one peak time zone is extracted from one average heart rate data. That is, when it is assumed that the time length of one piece of heart rate data is one day, the number of times where the meal is entirely performed is not one. That is, it is indisputable that the number of times where the peak time zone is extracted varies according to the number of times where the meal is actually performed. There may be cases where none of the peak time zones is extracted and the case where a plurality of peak time zones are extracted. In FIG. 4, the case where the time when the average heart rate decreases to the same value as the average heart rate measured at the start time of the peak time zone is set as the end time of the peak time zone is exemplified. However, the method for setting the end time of the peak time zone is not limited thereto. For example, the time at which the minimum value is first detected at the passage of time from the time when the maximum value is measured in the section satisfying the above condition can be set as the end time of the peak time zone.

The first detection unit 150 is a processing unit that detects the second peak at least partially overlapping the peak time zone from each heart rate data.

In one embodiment, the first detection unit 150 detects the second peak of each heart rate data acquired by the acquisition unit 120 for each peak time zone extracted by the extraction unit 140. That is, the first detection unit 150 selects one peak time zone from the peak time zones extracted by the extraction unit 140. Furthermore, the first detection unit 150 selects one piece of heart rate data from the heart rate data pieces acquired by the acquisition unit 120. In addition, as illustrated in the graph on the right portion of FIG. 3, the first detection unit 150 detects a period including a peak time zone and detects the period of the heart rate data satisfying the two conditions that the shape of the waveform is similar to the waveform of the peak time zone in the average heart rate data as the second peak zone. Here, as an example, a case where the second peak period, that is, the start time and the end time of the second peak period is input from the first detection unit 150 to the second detection unit 160 is exemplified. However, the measurement time of the second peak at which the heart rate of the second peak may be input.

More specifically, the first detection unit 150 extracts candidates of the second peak period using the same logic as that of the method of extracting the peak time zone by the extraction unit 140. Thereafter, the first detection unit 150 further extracts a candidate having a candidate including the previously selected peak time zone among the previously extracted second peak period candidates. Subsequently, the first detection unit 150 calculates the Euclidean distance at each time when the waveforms overlap each other between a partial waveform corresponding to the candidate of the second peak period including the peak time zone among the waveforms of the previously selected heart rate data and a partial waveform corresponding to the previously selected peak time zone among the waveforms of the average heart rate data. In a case where the sum of the Euclidean distances at each time is equal to or less than the predetermined threshold value, the first detection unit 150 detects the candidate of the second peak period including the peak time zone as the second peak period. In a case where there are no candidates satisfying the above two conditions among the candidates of the second peak period, the second peak period is not detected.

Here, as an example, it is assumed that a condition that the candidate of the second peak period includes the peak time zone is extracted as the second peak period. However, the candidate of the second peak period in which the period overlapping with the peak time zone is equal to or greater than the predetermined threshold value may be extracted. In addition, here, a case of determining whether or not the shape is similar to the waveform of the peak time zone in the average heart rate data based on whether or not the sum of Euclidean distances at each time is equal to or less than the threshold value is exemplified. However, similarity of the shape may be determined by another method. For example, it is also possible to determine the similarity of shapes based on similarity calculated between partial waveforms of each other, for example, whether or not the correlation coefficient is equal to or greater than the threshold value.

The second detection unit 160 is a processing unit that detects the first peak appearing prior to the second peak from each heart rate data.

As illustrated in the graph on the right portion of FIG. 3, the second detection unit 160 detects a peak of which the heart rate increasing width is equal to or greater than a predetermined threshold value as the first peak at the start time of the second peak period or within a predetermined period backward from the measurement time of the second peak where the heart rate of the second peak is measured on the waveform of the heart rate data selected by the first detection unit 150. While this does not fall within the scope of the claimed invention, it is indisputable that that the second detection unit 160 can detect the first peak also by other methods. For example, the second detection unit 160 can detect a peak occurring one time before the measurement time of the second peak detected by the first detection unit 150 as the first peak.

The estimation unit 170 is a processing unit that estimates the meal time from each heart rate data.

In one embodiment, the estimation unit 170 estimates the time when the minimum value is first detected backward from the measurement time of the first peak detected by the second detection unit 160, that is, the start time of the first peak period as the "meal start time". Furthermore, the estimation unit 170 estimates the measurement time of the first peak detected by the second detection unit 160 as the "meal end time". Furthermore, the estimation unit 170 estimates the "meal turnaround time" by subtracting the meal start time from the meal end time. At least one of the meal start time, the meal end time, the meal turnaround time, or a combination thereof estimated as described above is output to the service provision unit 180.

The service provision unit 180 is a processing unit that provides the above-described healthcare supporting service.

In one embodiment, the service provision unit 180 records at least one of the meal time, for example, the meal start time, the meal end time, the meal turnaround time, or the combination thereof, generates the list of the meal time zones over a predetermined period, for example, one week or the like from the meal time recorded so far and outputs the generated list, or analyzes eating habits or diet from the meal time recorded so far and outputs various advices.

The processing units such as the acquisition unit 120, the calculation unit 130, the extraction unit 140, the first detection unit 150, the second detection unit 160, the estimation unit 170, and the service provision unit 180 can be implemented as follows. For example, it can be realized such that a process that exhibits the same function as the acquisition unit 120, the calculation unit 130, the extraction unit 140, the first detection unit 150, the second detection unit 160, the estimation unit 170, and the service provision unit 180 is caused by the central processing device such as the CPU to develop on the memory and execute the process. These processing units are not entirely executed by the central processing unit and may be executed by the MPU. In addition, each of the above functional units can also be realized by hard-wired logic.

In addition, various semiconductor memory devices, for example, the RAM and flash memory can be adopted as the main storage device used by each of the above processing units. In addition, the storage device referred to by each of the functional units described above is not entirely a main storage device and may be an auxiliary storage device. In this case, the HDD, the optical disk, the SSD, or the like can be adopted.

### [Flow of Processing]

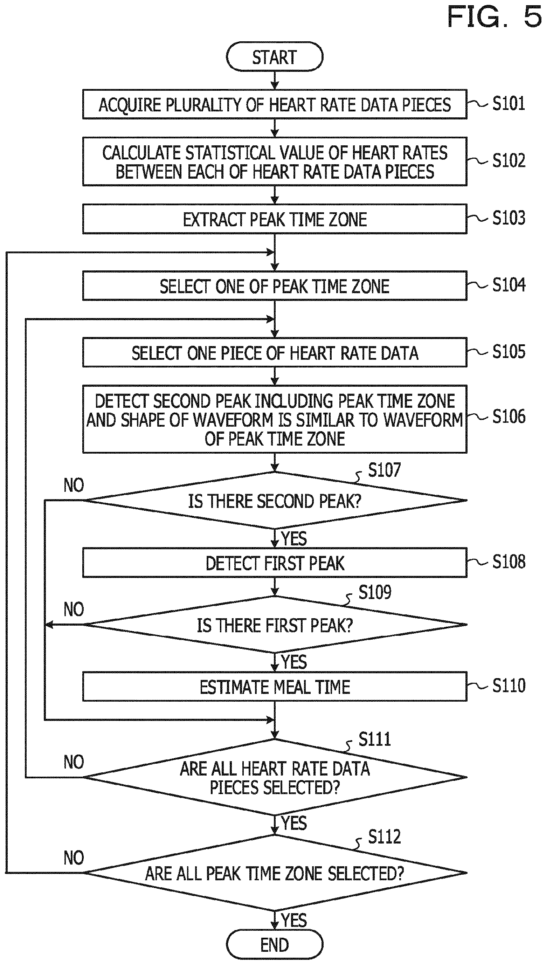

FIG. 5 is a flowchart illustrating a procedure of meal time estimation processing according to Example 1. This processing is started, for example, in a case where a plurality of heart rate data pieces, for example, the heart rate data items such as three days, one week, one month, and the like are accumulated in the sensor terminal 10 or the information processing device 100 or the like.

As illustrated in FIG. 5, in a case where a plurality of heart rate data pieces are acquired (step S101), the calculation unit 130 averages the heart rates measured at the same time among each heart rate data, and calculates the heart rate data (step S102).

In addition, the extraction unit 140 extracts the section where the increasing width H of the heart rate data on the waveform of the average heart rate data obtained in step S102 is equal to or greater than that the first threshold value Th_{H} and the rising period W of the heart rate is equal to or more than the second threshold value Thw as a peak time zone (step S103).

The first detection unit 150 selects one peak time zone out of the peak time zones extracted in step S103 (step S104). Furthermore, the first detection unit 150 selects one piece of heart rate data from the heart rate data acquired in step S101 (step S105).

In addition, the first detection unit 150 detects a period including a peak time zone selected in step S104 and the period of the heart rate data in which the shape of the waveform is similar to the waveform of the peak time zone in the average heart rate data as the second peak zone (step S106).

At this time, in a case where the detection of the second peak succeeds in step S106 (Yes in step S107), the second detection unit 160 detects a peak of which the heart rate increasing width is equal to or greater than a predetermined threshold value as the first peak at the start time of the second peak period or within a predetermined period backward from the measurement time of the second peak where the heart rate of the second peak is measured on the waveform of the heart rate data selected in step S105 (step S108). In a case where the detection of the second peak is not successful (No in step S107), the process proceeds to step S111.

Here, in a case where the first peak is successfully detected in step S108 (Yes in step S109), the estimation unit 170 estimates at least one of the "meal start time", the "meal end time", the "meal turnaround time", and the combination thereof from the first peak detected in step S108 as the "meal time" (step S110). In a case where the detection of the first peak is not successful (No in step S109), the process also proceeds to step S111.

Until all the heart rate data pieces acquired in step S101 are selected (No in step S111), the processes in steps S105 to S110 are repeatedly executed. Thereafter, in a case where all the heart rate data pieces acquired in step S101 are selected (Yes in step S111), the process proceeds to step S112. Until all the peak time zones extracted in step S103 are selected (No in step S112), the processes in steps S104 to S111 are repeatedly executed. Finally, in a case where all the peak time zones extracted in step S103 are selected (Yes in step S112), the process is ended.

### [One Aspect of Effect]

As described above, the information processing device 100 according to the present example realizes meal time estimation processing for detecting the first peak from the second peak of the time series data of each heart rate overlapping with the peak time zone appearing in the waveform of the average heart rate data obtained by averaging the plurality of time series data pieces of the heart rate data for each time and estimating the meal time. Therefore, according to the information processing device 100 according to the present example, as one aspect, it is possible to suppress a decrease in estimation accuracy of the meal time even when supervised data is not applied as an input.

### [Example 2] (comparative example not falling within the scope of the claimed invention)

Meal time estimation may be implemented in various different forms in addition to the embodiments described above. Therefore, another example will be described below This example does not fall within the scope of the claimed invention.

### [Generation of Meal Estimation Model]

For example, the information processing device 100 can also perform machine learning a "meal estimation model" that that classifies feature amount relating to the meals derived from heart rate data given as input into either a meal or non-meal class using the labeled supervised data generated from the each heart rate data and the estimation result of the meal time for each heart rate data. For such machine learning, an arbitrary algorithm such as support vector machine, boosting, neural network, and the like can be adopted as an example.

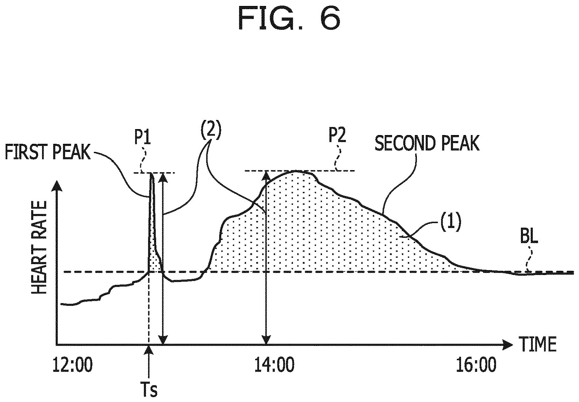

The arbitrary feature amount can be adopted as the above-mentioned feature amount. An example thereof will be described with reference to FIG. 6. FIG. 6 is a diagram illustrating an example of the heart rate data. In FIG. 6, the changes in the heart rate before and after the start of the meal are graphed. The vertical axis illustrated in FIG. 6 represents the heart rate per unit time and a horizontal axis represents an elapsed time (time) from immediately before the meal starts. "BL" illustrated in FIG. 6 represents a baseline of the heart rate. The baseline BL is a reference value for obtaining an increase in the heart rate caused by the meals. The reason why the base line is the reference value is that since the heart rate does not entirely have the same value at each of the scenes at which the user eats, by obtaining the feature amount using the increasing width of the heart rate instead of a modulus of the heart rate, it is used to suppress the deterioration of the estimation precision of the meal time due to the variation in the heart rate in each scene in which the meal is ingested. For example, the heart rate at the meal start time Ts can be used as a value of the heart rate of the baseline BL. In addition to this, the average value of the heart rates for a predetermined period such as 30 minutes or 1 hour before starting the meal of the user, the heart rate of the meal start time Ts, and the minimum heart rate between the first peak and the second peak.

For example, from the viewpoints of (1) the area, (2) the amplitude, and the like, it is possible to define a feature amount representing likelihood that the cause of change in heart rate is the meal. Although two feature amounts are exemplified here, it may not entirely to use all the feature amounts for estimating the meal time and it is possible to calculate the feature vector by further combining at least one of the two feature amounts or another feature amount.

That is, as illustrated in FIG. 6, the area of the first peak region A1 and the area of the second peak region A2 are defined as the feature amount (1). In addition, as illustrated in FIG. 6, a maximum heart rate P1 at which the heart rate takes the maximum value among the waveforms forming the first peak and a maximum heart rate P2 at which the heart rate takes the maximum value among the waveforms forming the second peak are defined as the feature amounts (2). Here, a calculation method of the above two feature amounts will be described.

Among the feature amount (1), an area S1 of the first peak region A1 can be obtained by summing the increasing widths of the heart rate from the baseline BL in a meal period Ta1 starting from the meal start time Ts and ending at the time when the heart rate recovered to the baseline BL via the first peak. Although the case of calculating the area by summing up the increasing widths of the heart rate is exemplified here, it may be also possible to calculate the average value of the increasing width of the heart rate. On the other hand, as an example, an area S2 of the second peak region A2 can be obtained by summing the increasing widths of the heart rate from the baseline BL in a after meal period Ta2 ending from the meal start time Ts and ending at the time when the heart rate recovered to the baseline BL via the second peak at an end point of the meal period Ta1 of the first peak region A1 or setting a time after than the end point at as the start point. Although the case of obtaining two areas is exemplified here, at least one of the feature amounts may be calculated without calculating the feature amounts.

The amplitude of the first peak among the above-described feature amounts (2) can be derived by extracting the maximum heart rate P1 at which the heart rate is the maximum among the heart rate measured in the above meal period Ta1 from the heart rate data. Similarly, the amplitude of the second peak can be derived by extracting the maximum heart rate P2 at which the heart rate is the maximum among the heart rate measured in the after meal period Ta2 from the heart rate data.

Under the definition of such feature amount, the feature amount is calculated from the heart rate data according to the following procedure. Specifically, the information processing device 100 cuts a part of the heart rate data as "window data" and calculates the feature amount for each window data. For example, the information processing device 100 sets a window having a predetermined time length, for example, 210 minutes, in the heart rate data. Subsequently, the information processing device 100 cuts out partial data corresponding to the section where the window is set. Thereafter, the information processing device 100 shifts the window set in the previous time over a predetermined shift width, for example, for 5 minutes or 30 minutes. The information processing device 100 cuts out the partial data corresponding to the window after the shifting. The window data is generated each time when the partial data is cut out with the window width as described above. In addition, the information processing device 100 can set the start time of the window data as a candidate for the meal start time for each window data, and calculate the feature amount relating to the area and the amplitude.

Here, in a case of generating labeled supervised data, the information processing device 100 uses the estimation result of the meal time estimated according to the flowchart illustrated in FIG. 5 for labeling. For example, the information processing device 100 generates a plurality of window data pieces from the heart rate data for each heart rate data for which the meal time estimation is executed, and calculates the feature vector for each window data. A label "meal" is attached to the feature vector of which the start time of the window data coincides with the estimated meal start time to generate positive data, and a label "non-meal" is attached to the feature vector of which the start time of the window data does not coincide with the estimated meal start time to generate negative data. In a case of generating negative data in this manner, from the viewpoint of generating the feature amount vector positioned near an identification boundary of the classification as negative data, the time zone of the window data for calculating the feature vector can be narrowed down in a predetermined period, for example, from 1 hour before starting of second peak to the end of the second peak. In this regard, it is possible to generate the labeled supervised data including positive data and negative data.

Thereafter, the information processing device 100 generates the meal estimation model by performing machine learning using the labeled supervised data generated as above. By using such a meal estimation model, the information processing device 100 estimates the meal time by classifying the feature vectors calculated for each window data from arbitrary heart rate data applied as input into a meal or non-meal class.

By these processes, it is possible to realize the estimation of the meal time by machine learning without manual labor assigning the labels to the supervised data. In the above example, a case where both positive data and negative data are used for machine learning is exemplified. However, only positive data may be used for machine learning.

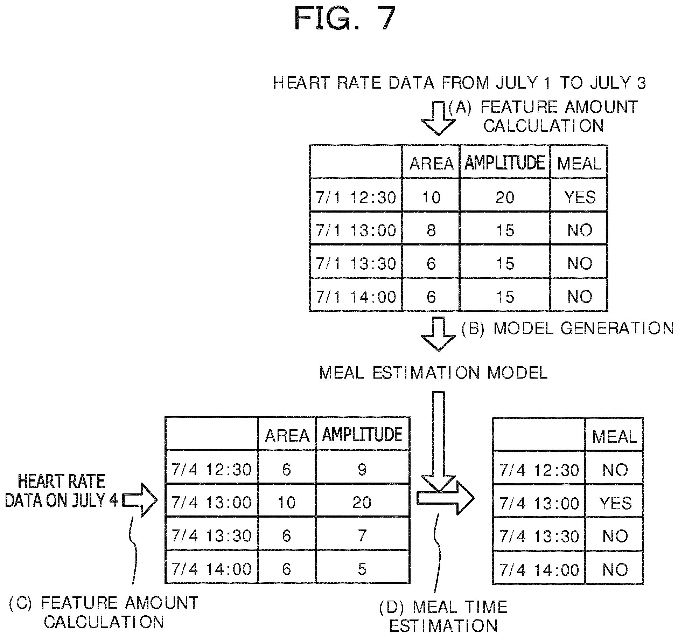

FIG. 7 is a diagram illustrating an example of a method for estimating the meal time by the machine learning. FIG. 7 illustrates an example in which the machine learning is performed by using the estimation result of the meal time of the heart rate data for 3 days from July 1 to July 3 to generate labeled supervised data, and the meal time is estimated from the heart rate data input after July 4 by using the meal estimation model obtained by machine learning.

As illustrated in FIG. 7, the information processing device 100 generates a plurality of window data pieces including one of window data using at least the meal start time of the estimation result as a starting point of a window from the heart rate data for each heart rate data for 3 days from July 1 to July 3 and calculates the feature vectors relating to each window data (A). Therefore, positive data and negative data are obtained as labeled supervised data. For example, in the illustrated example, the feature vectors of window data pieces starting from 12:30 on July 1, that is, data on the first line in FIG. 7 is generated as the positive data and the feature vectors of the window data pieces starting from 12:30, 13:00, 13:30, and 14:00 on July 1, that is, data pieces of second to fourth lines in FIG. 7 are generated as the negative data. In addition, the information processing device 100 generates a meal estimation model by performing machine learning using labeled supervised data generated as in (A) above (B).

Under the situation where the meal estimation model is generated in such a manner, when the heart rate data on July 4 is acquired, the information processing device 100 generates a plurality of window data pieces from the heart rate data, and calculates the feature vector for each window data (C). In addition, the information processing device 100 estimates the meal time by classifying the feature vector calculated as in (A) above into a meal or non-meal class using the above-described meal estimation model. For example, in this example, since the feature vector of the window data starting at 13:00 on July 4 is classified as "meal", as an example of the estimation result of the meal time, the meal start time "13:00" is output.

In addition, it is possible to estimate the index related to meal using the feature vector of the feature vector of the positive data and the feature vector classified into the class of the meal. The indicator related to meals is a human condition, eating behavior, caloric intake, or the like. Since the index relating to the meal can be expressed as a function of the feature vector, in a case where the feature vector is defined as x, the index relating to the meal can be expressed as f(x). As an example, when the index relating to the meal is set as the calorie intake, the area of the second peak can be used as x.

### [Stand-alone]

In Example 1 above, a case where it is constructed as a client server system including the sensor terminals 10 and the information processing device 100 is exemplified. However, the embodiments are not limited thereto. For example, a series of processing from acquisition of the heart rate data to the estimation of meal time may be performed by the sensor terminals 10, the information processing device 100, or other computers in a stand-alone manner.

### [Application Example of System]

In Example 1 above, the healthcare supporting system 1 includes the information processing device 100. However, the healthcare supporting system 1 may not include the information processing device 100. That is, in a case where the sensor terminal 10 is mounted as the wearable gadget or the like, various processing other than acquisition of the heart rate data, such as estimation of meal times is executed by the smartphone or the tablet terminal coupled by the near field communication or the like by the wearable gadget.

### [Distribution and Integration]

In addition, each configuration element of the illustrated apparatus is not entirely and physically configured as illustrated in the drawing. That is, the specific aspect of distribution and integration of each device is not limited to those illustrated in the drawing, and all or a part thereof may be distributed functionally or physically in arbitrary units according to various loads and usage conditions or the like. For example, the acquisition unit 120, the calculation unit 130, the extraction unit 140, the first detection unit 150, the second detection unit 160, the estimation unit 170, or the service provision unit 180 may be coupled as an external device of information processing device 100 via a network. In addition, another device includes the acquisition unit 120, the calculation unit 130, the extraction unit 140, the first detection unit 150, the second detection unit 160, the estimation unit 170, and the service provision unit 180. The functions of the above information processing device 100 may be realized in corporation with the units with each other through network connection.

### [Meal Time Estimation Program]

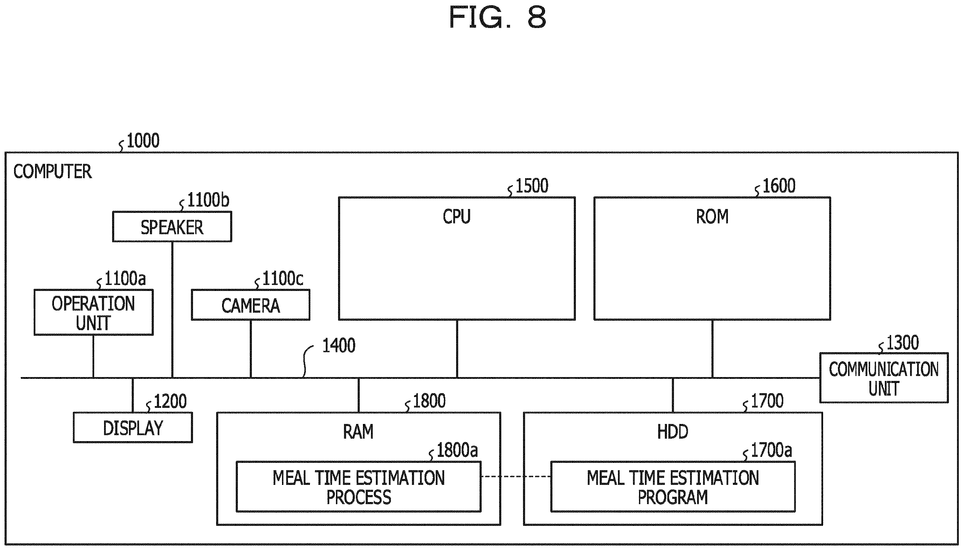

In addition, the various processing described in the above embodiments can be realized by executing a prepared program on the computer such as a personal computer or a workstation. Therefore, hereinafter, an example of the computer that executes the meal time estimation program having the same function as in the above embodiment will be described with reference to FIG. 8.

FIG. 8 is a diagram illustrating a hardware configuration example of a computer that executes meal time estimation program according to Example 1 and Example 2. As illustrated in FIG. 8, the computer 1000 includes an operation unit 1100a, a speaker 1100b, a camera 1100c, a display 1200, and a communication unit 1300. Furthermore, the computer 1000 includes a CPU 1500, a ROM 1600, an HDD 1700, and a RAM 1800. The respective units 1100 to 1800 are coupled via a bus 1400.

As illustrated in FIG. 8, a meal time estimation program 1700a that exhibits the same function as the acquisition unit 120, the calculation unit 130, the extraction unit 140, the first detection unit 150, the second detection unit 160, the estimation unit 170, and the service provision unit 180 illustrated in Example 1 above is stored in the HDD 1700. Similar to each configuration component, the meal time estimation program 1700a may be integrated or separated as with the respective configuration components of the acquisition unit 120, the calculation unit 130, the extraction unit 140, the first detection unit 150, the second detection unit 160, the estimation unit 170, and the service provision unit 180 illustrated in FIG. 1. That is, it is not entirely desire for all data pieces illustrated in Example 1 above to be stored in the HDD 1700, and data to be used for processing may be stored in the HDD 1700.

Under such circumstances, the CPU 1500 reads the meal time estimation program 1700a from the HDD 1700 and develops the program in the RAM 1800. As a result, the meal time estimation program 1700a function as the meal time estimation process 1800a as illustrated in FIG. 8. The meal time estimation process 1800a expands various data pieces read from the HDD 1700 into the region allocated to the meal time estimation process 1800a in the storage area of the RAM 1800 and executes various processing using the developed various data pieces. For example, as an example of the processing executed by the meal time estimation process 1800a, the processing illustrated in FIG. 5 and the like are included. In the CPU 1500, all the processing units illustrated in Example 1 do not entirely operate, and it suffices that the processing unit corresponding to the processing to be executed is virtually realized.

The meal time estimation program 1700a may not entirely be stored in the HDD 1700 or the ROM 1600 from the beginning. For example, each program is stored in a "portable physical medium" such as a flexible disk inserted in the computer 1000, so-called FD, CD-ROM, DVD disk, magnetooptical disk, IC card, or the like. The computer 1000 may obtain and execute each program from these portable physical media. In addition, each program may be stored in another computer or server device coupled to the computer 1000 via a public line, the Internet, a LAN, a WAN, or the like. Therefore, the computer 1000 acquires and executes each program from theses.

### [Reference Signs List]

1: healthcare supporting system
10: sensor terminal
11: heart rate data acquisition unit
13: communication I/F unit
100: information processing device
110: communication I/F unit
120: acquisition unit
130: calculation unit
140: extraction unit
150: first detection unit
160: second detection unit
170: estimation unit
180: service provision unit

## Claims

1. An information processing apparatus (1000) comprising:
a memory (1600, 1700, 1800); and
a processor (1500) coupled to the memory and configured to:
acquire from the memory (1600, 1700, 1800) time series data representing heart rate as a function of time for each of a plurality of days and for the same person,
apply statistical processing to the time series data to obtain a waveform of average heart rate data representing statistical heart rate for the same period of time of the plurality of days,
extract, from the waveform representing statistical heart rate, a peak time zone in which a width of the average heart rate data on the waveform representing statistical heart rate increases to be equal to or greater than a first threshold value and a rising period of the heart rate is equal to or larger than a second threshold value,
detect, in a waveform of time series data of a given day, a second time zone that includes the peak time zone and has a shape similar to the waveform of the peak time zone,
detect, in the waveform of time series data of the given day, a peak in which a width of the time series data representing heart rate on the waveform of time series data of the given day increases to be equal to or greater than a predetermined threshold, the peak occurring at the start time of the second time zone or within a predetermined period before the start of the second time zone, the peak representing an estimated meal time, and
output the estimated meal time.

2. The apparatus (1000) according to claim 1, wherein
the meal time includes at least one of a meal start time, a meal end time, and a meal turnaround time.

3. A method executed by a computer, the method comprising:
acquiring (S101) from a memory time series data representing heart rate as a function of time for each of a plurality of days and for the same person;
applying (S102) statistical processing to the time series data to obtain a waveform of average heart rate data representing statistical heart rate for the same period of time of the plurality of days;
extracting (S103), from the waveform representing statistical heart rate, a peak time zone in which a width of the average heart rate date on the waveform representing statistical heart rate increases to be equal to or greater than a first threshold value and a rising period of the heart rate is equal to or larger than a second threshold value;
detecting (S106), in a waveform of time series data of a given day, a second time zone that includes the peak time zone and has a shape similar to the waveform of the peak time zone;
detecting (S108), in the waveform of time series data of the given days, a peak in which a width of the time series data representing heart rate on the waveform of time series data of the given day increases to be equal to or greater than a predetermined threshold, the peak occurring at the start time of the second time zone or within a predetermined period before the start of the second time zone, the peak representing an estimated meal time; and
outputting the estimated meal time.

4. A program which when executed by a processor, causes the processor to perform the method of claim 3.

## Patentansprüche

1. Informationsverarbeitungsvorrichtung (1000), umfassend:
einen Speicher (1600, 1700, 1800); und
einen Prozessor (1500), der mit dem Speicher verbunden ist und eingerichtet ist zum:
Erlangen, aus dem Speicher (1600, 1700, 1800), von Zeitreihendaten, die eine Herzfrequenz als Funktion der Zeit für jeden von mehreren Tagen und für dieselbe Person darstellen,
Anwenden einer statistischen Verarbeitung auf die Zeitreihendaten, um eine Wellenform von durchschnittlichen Herzfrequenzdaten zu erhalten, die eine statistische Herzfrequenz für dieselbe Zeitperiode der mehreren Tagen darstellt,
Extrahieren, aus der Wellenform, die die statistische Herzfrequenz darstellt, eines Spitzenzeitbereichs, in dem eine Breite der durchschnittlichen Herzfrequenzdaten auf der Wellenform, die die statistische Herzfrequenz darstellt, zunimmt, um gleich oder größer als ein erster Schwellenwert zu sein, und eine Anstiegsperiode der Herzfrequenz gleich oder größer als ein zweiter Schwellenwert ist,
Erkennen, in einer Wellenform von Zeitreihendaten eines gegebenen Tages, eines zweiten Zeitbereichs, der den Spitzenzeitbereich aufweist und eine ähnliche Form hat wie die Wellenform des Spitzenzeitbereichs,
Erkennen, in der Wellenform von Zeitreihendaten des gegebenen Tages, einer Spitze, bei der eine Breite der Zeitreihendaten, die die Herzfrequenz auf der Wellenform von Zeitreihendaten des gegebenen Tages darstellen, zunimmt, um gleich oder größer als ein vorbestimmter Schwellenwert zu sein, wobei die Spitze zur Startzeit des zweiten Zeitbereichs oder innerhalb einer vorbestimmten Periode vor dem Start des zweiten Zeitbereichs auftritt, wobei die Spitze eine geschätzte Essenszeit darstellt, und
Ausgeben der geschätzten Essenszeit.

2. Vorrichtung (1000) nach Anspruch 1, wobei
die Essenszeit mindestens eine von einer Essensstartzeit, einer Essensendezeit und einer Essensdurchlaufzeit aufweist.

3. Verfahren, das von einem Computer ausgeführt wird, wobei das Verfahren umfasst:
Erlangen (S101), aus einem Speicher, von Zeitreihendaten, die die Herzfrequenz als Funktion der Zeit für jeden von mehreren Tagen und für dieselbe Person darstellen;
Anwenden (S102) einer statistischen Verarbeitung auf die Zeitreihendaten, um eine Wellenform von durchschnittlichen Herzfrequenzdaten zu erhalten, die eine statistische Herzfrequenz für dieselbe Zeitperiode der mehreren Tage darstellen;
Extrahieren (S103), aus der Wellenform, die die statistische Herzfrequenz darstellt, eines Spitzenzeitbereichs, in dem eine Breite der durchschnittlichen Herzfrequenzdaten auf der Wellenform, die die statistische Herzfrequenz darstellt, zunimmt, um gleich oder größer als ein erster Schwellenwert zu sein, und eine Anstiegsperiode der Herzfrequenz gleich oder größer als ein zweiter Schwellenwert ist;
Erkennen, (S106), in einer Wellenform von Zeitreihendaten eines gegebenen Tages, eines zweiten Zeitbereichs, der den Spitzenzeitbereich aufweist und eine ähnliche Form hat wie die Wellenform des Spitzenzeitbereichs;
Erkennen (S108), in der Wellenform von Zeitreihendaten des gegebenen Tages, einer Spitze, bei der eine Breite der Zeitreihendaten, die die Herz-frequenz auf der Wellenform von Zeitreihendaten des gegebenen Tages darstellen, zunimmt, um gleich oder größer als eine vorbestimmte Schwelle zu sein, wobei die Spitze zur Startzeit des zweiten Zeitbereichs oder innerhalb einer vorbestimmten Zeitperiode vor dem Start des zweiten Zeitbereichs auftritt,
wobei die Spitze eine geschätzte Essenszeit darstellt; und
Ausgeben der geschätzten Essenszeit.

4. Programm, das, wenn es von einem Prozessor ausgeführt wird, den Prozessor veranlasst, das Verfahren nach Anspruch 3 durchzuführen.

## Revendications

1. Appareil de traitement d'informations (1000) comprenant :
une mémoire (1600, 1700, 1800); et
un processeur (1500) couplé à la mémoire et configuré pour :
acquérir à partir de la mémoire (1600, 1700, 1800) des données de séries chronologiques représentant la fréquence cardiaque en fonction du temps pour chacun d'une pluralité de jours et pour la même personne,
appliquer un traitement statistique aux données de séries chronologiques pour obtenir une forme d'onde de données de fréquence cardiaque moyenne représentant la fréquence cardiaque statistique pour la même période de temps de la pluralité de jours, extraire, à partir de la forme d'onde représentant la fréquence cardiaque statistique, un fuseau horaire de pointe dans lequel une largeur des données de fréquence cardiaque moyenne sur la forme d'onde représentant la fréquence cardiaque statistique augmente afin d'être égale ou supérieure à une première valeur de seuil et une période de montée de la fréquence cardiaque est égale ou supérieure à une deuxième valeur de seuil, détecter, dans une forme d'onde de données de séries temporelles d'un jour donné, un deuxième fuseau horaire qui inclut le fuseau horaire de pointe et a une forme similaire à la forme d'onde du fuseau horaire de pointe,
détecter, dans la forme d'onde des données de séries temporelles du jour donné, un pic dans lequel une largeur des données de séries temporelles représentant la fréquence cardiaque sur la forme d'onde des données de séries temporelles du jour donné augmente afin d'être égale ou supérieure à un seuil prédéterminé, le pic se produisant à l'heure de début du deuxième fuseau horaire ou dans une période prédéterminée avant le début du deuxième fuseau horaire, le pic représentant une heure de repas estimée, et
sortir l'heure de repas estimée.

2. Appareil (1000) selon la revendication 1, dans lequel l'heure du repas comprend au moins une heure de début de repas, une heure de fin de repas et une heure d'exécution de repas.

3. Procédé exécuté par un ordinateur, le procédé comprenant de :
acquérir (S101), à partir d'une mémoire, des données de séries temporelles représentant la fréquence cardiaque en fonction du temps pour chacun d'une pluralité de jours et pour la même personne ;
appliquer (S102) un traitement statistique aux données de séries temporelles pour obtenir une forme d'onde de données de fréquence cardiaque moyenne représentant la fréquence cardiaque statistique pour la même période de temps de la pluralité de jours ;
extraire (S103), à partir de la forme d'onde représentant la fréquence cardiaque statistique, une plage horaire maximale dans laquelle une largeur de la date de fréquence cardiaque moyenne sur la forme d'onde représentant la fréquence cardiaque statistique augmente afin d'être égale ou supérieure à une première valeur de seuil et une période de montée de la fréquence cardiaque est égale ou supérieure à une deuxième valeur de seuil ;
détecter (S106), dans une forme d'onde de données de séries chronologiques d'un jour donné, un second fuseau horaire qui inclut le fuseau horaire de pointe et a une forme similaire à la forme d'onde du fuseau horaire de pointe ;
détecter (S108), dans la forme d'onde des données de séries temporelles des jours donnés, un pic dans lequel une largeur des données de séries temporelles représentant la fréquence cardiaque sur la forme d'onde des données de séries temporelles du jour donné augmente pour être égale ou supérieure à un seuil prédéterminé, le pic se produisant à l'heure de début du second fuseau horaire ou dans une période prédéterminée avant le début du second fuseau horaire, le pic représentant une heure de repas estimée ; et
sortir l'heure de repas estimée.

4. Programme qui, lorsqu'il est exécuté par un processeur, amène le processeur à exécuter le procédé de la revendication 3.
